# EUROPÄISCHE PATENTSCHRIFT

(11) **EP 4 151 249 B1**
(45) Veröffentlichungstag und Bekanntmachung des Hinweises auf die Patenterteilung: **10.09.2025**
(21) Anmeldenummer: 22195884.6
(22) Anmeldetag: 15.09.2022
(51) Int. Cl.: A61M 1/00, A61F 9/007, A61M 3/02

(54) **VERFAHREN ZUM BETREIBEN EINER FLUIDPUMPE UND OPHTHALMOCHIRURGISCHES SYSTEM HIERMIT**
METHOD FOR OPERATING A FLUID PUMP AND OPHTHALMOSURGICAL SYSTEM THEREWITH
PROCÉDÉ DE FONCTIONNEMENT D'UNE POMPE À FLUIDE ET SYSTÈME DE CHIRURGIE OPHTALMOLOGIQUE L'UTILISANT

(30) Priorität: 21.09.2021 DE 102021124415
(43) Veröffentlichungstag der Anmeldung: 22.03.2023
(73) Patentinhaber: Carl Zeiss Meditec AG, 07745 Jena (DE)
(72) Erfinder: Kuebler, Christoph, 73447 Oberkochen (DE); Kohlhammer, Susanne, 89134 Blaustein (DE)
(74) Vertreter: Hofstetter, Schurack & Partner

(56) Entgegenhaltungen:
- DE-A1- 3 716 765
- DE-B3- 102016 201 297
- US-A1- 2013 303 978
- US-A1- 2020 353 133
- US-A1- 2021 093 482

## Beschreibung

Die Erfindung betrifft eine Kassette für eine Konsole eines ophthalmochirurgischen Systems zur Behandlung eines Auges, wobei die Kassette zum Einsetzen in einen Kassettenaufnahmebereich der Konsole ausgebildet ist und wenigstens eine Fluidpumpe zum Fördern eines Behandlungsfluids aufweist, wobei die Fluidpumpe eine Pumpkammer und eine mittels eines zumindest bereichsweise auslenkbaren Trennelements von der Pumpkammer getrennte Antriebskammer aufweist, wobei der Antriebskammer ein Antriebsfluid und der Pumpkammer das Behandlungsfluid zuführbar ist. Ferner umfasst die Erfindung eine Konsole eines ophthalmochirurgischen Systems zur Behandlung eines Auges, zumindest mit einem Kassettenaufnahmebereich zum Aufnehmen einer Kassette, die wenigstens eine Fluidpumpe zum Fördern eines Behandlungsfluids aufweist, wobei die Fluidpumpe eine Pumpkammer und eine mittels eines zumindest bereichsweise auslenkbaren Trennelements von der Pumpkammer getrennte Antriebskammer aufweist, wobei der Antriebskammer ein Antriebsfluid und der Pumpkammer das Behandlungsfluid zuführbar ist, einem Antriebsfluidversorgungssystem zum Zuführen des Antriebsfluids in die Antriebskammer, und einer Sensoreinheit zum Erfassen einer Auslenkungsposition des Trennelements. Schließlich betrifft die Erfindung auch ein ophthalmochirurgisches System zur Behandlung eines Auges.

Ophthalmochirurgische Systeme, Konsolen hierfür sowie Kassetten zum Einsetzen in Konsolen sind im Stand der Technik bekannt, sodass es diesbezüglich eines gesonderten druckschriftlichen Nachweises dem Grunde nach nicht bedarf. Zur Behandlung einer Augenlinsentrübung, in der Medizin auch als grauer Star bezeichnet, sind unterschiedliche chirurgische Techniken bekannt. Am weitesten verbreitet ist die Phakoemulsifikation, bei der eine dünne Hohlnadel in einen Kapselsack, in dem die Augenlinse angeordnet ist, eingeführt und zu Ultraschallschwingungen angeregt wird. Mittels der vibrierenden Hohlnadel kann die Linse emulsifiziert werden, wobei hierbei freigesetzte Linsenpartikel über eine Aspirationsleitung mittels einer Pumpe abgesaugt werden können. Dabei wird ein Spülfluid, auch Irrigationsfluid genannt, zugeführt. Die Linsenpartikel werden zusammen mit dem Fluid als Aspirationsfluid abgesaugt. Sobald die Linse vollständig emulsifiziert und entfernt ist, kann in den dann geleerten Kapselsack eine neue künstliche Linse eingesetzt werden. Hierdurch kann für den behandelten Patienten wieder ein gutes Sehvermögen erreicht werden.

Ein fortschrittliches ophthalmochirurgisches System, welches sich für die Phakoemulsifikation als besonders geeignet herausgestellt hat, offenbart zum Beispiel die DE 10 2016 201 297 B3. Bei diesem System werden jeweils zwei strömungstechnisch parallelgeschaltete Fluidpumpen für die Irrigation sowie auch für die Aspiration genutzt. Jede der Fuidpumpen weist eine Pumpkammer und eine mittels eines Trennelements von der Pumpkammer getrennte Antriebskammer auf. Die Antriebskammer wird für den bestimmungsgemäßen Betrieb der Fluidpumpe mit einem Antriebsfluid beaufschlagt, dessen Antriebsdruck zur Ausführung eines jeweiligen Pumphubs variiert wird. Dadurch verändert sich abhängig hiervon eine Auslenkungsposition des Trennelements, die sich entsprechend auf die Pumpkammer auswirkt. Die Pumpkammer ist mit dem jeweiligen Behandlungsfluid, beispielsweise dem Irrigationsfluid, dem Aspirationsfluid oder dergleichen, beaufschlagt. Durch geeignetes Steuern eines Einlass- und eines Auslassventils der Fluidpumpe, kann dann die Förderwirkung erreicht werden.

Eine Auslenkungsposition des Trennelements wird mittels eines der jeweiligen Fluidpumpe zugeordneten Auslenkungspositionssensors erfasst. Eine Steuereinrichtung des ophthalmochirurgischen Systems, insbesondere der Konsole steuert die Funktion der Fluidpumpe zumindest abhängig von einem Sensorsignal des Auslenkungspositionssensors und einem mittels eines Antriebsdrucksensors bereitgestellten Antriebsdrucksignal. Die Steuereinrichtung kann ergänzend beispielsweise das Einlass- und das Auslassventil entsprechend steuern.

Durch wechselseitiges Betätigen der jeweils zwei parallelgeschalteten Fluidpumpen kann während einer Operation ein Volumenstrom mit sehr geringen Schwankungen erreicht werden. Dadurch kann sich ein nahezu konstanter Augeninnendruck im Kapselsack einstellen. Solange ausreichend Irrigationsfluid zugeführt werden kann, kann das System auch während einer sehr lang andauernden Operation nahezu ohne Unterbrechung des Irrigationsfluidstroms betrieben werden.

Das Trennelement einer jeweiligen der Fluidpumpen wird somit nicht durch einen Stößel oder eine Stange betätigt, sondern vielmehr durch das Antriebsfluid. Dadurch kann eine nahezu ruckfreie und sehr schnelle Betätigung erreicht werden. Zugleich erweist sich die auf diese Weise realisierte Fluidpumpe als sehr wartungsarm und zuverlässig. Das mit der DE 10 2016 201 297 B3 offenbarte ophthalmochirurgische System eignet sich daher besonders auch für eine Verfahrensführung, die eine Reaktion bei einem Verstopfen einer Nadelspitze der Hohlnadel beziehungsweise der Absaugöffnung erfordert. Ein solcher Zustand wird auch als Okklusion bezeichnet. Mit dem in der DE 10 2016 201 297 B3 vorgestellten ophthalmochirurgischen System kann auch diese Situation sehr gut gehandhabt werden, sodass ein Augeninnendruck auch bei solchen Störungen im Betrieb nahezu konstant gehalten werden kann.

Für den bestimmungsgemäßen Betrieb des ophthalmochirurgischen Systems ist es erwünscht, durch Regelung des Drucks des Irrigationsfluids und des Vakuums des Aspirationsfluids den Augeninnendruck, insbesondere im Kapselsack, möglichst konstant zu halten. Hierzu ist es erwünscht, den Druck des Behandlungsfluids möglichst genau zu kennen, damit eine entsprechend genaue Regelung dieses Drucks erreicht werden kann.

Bei der zuvor beschriebenen Fluidpumpe hängt der Druck des Behandlungsfluids vom Antriebsdruck des Antriebsfluids ab. Darüber hinaus besteht auch eine Abhängigkeit von Eigenschaften des Trennelements. Aufgrund von Bauteilschwankungen und Toleranzen sowie Alterungseffekten durch Lagerung oder dergleichen treten Abweichungen der Eigenschaften zwischen unterschiedlichen Trennelementen auf. Diese Abweichungen können zum Beispiel eine Abhängigkeit einer durch das Trennelement bewirkten Druckdifferenz von einer jeweiligen Auslenkungsstellung des Trennelements betreffen. Eine solche Abweichung kann über einem gewünschten Genauigkeitsbereich liegen, der für die Regelung des Augeninnendrucks zweckmäßig ist. Insgesamt ist es wünschenswert, die Auslenkungsposition des Trennelements möglichst genau zu kennen, um daraus unter anderem den Druck des Behandlungsfluids ermitteln zu können. In diesem Zusammenhang offenbart die DE 37 16 765 A1 eine Membran mit Magnet. Ferner offenbart die US 2004/0265150 A1 ein Magnetmembransystem.

Es ist daher die Aufgabe der Erfindung, eine Kassette, eine Konsole sowie ein ophthalmochirurgisches System dahingehend zu verbessern, dass die Auslenkungsposition des Trennelements insbesondere zum Regeln in Bezug auf die Behandlungsflüssigkeit besser erfasst werden kann.

Als Lösung werden mit der Erfindung eine Kassette für eine Konsole eines ophthalmochirurgischen Systems zur Behandlung eines Auges, eine Konsole eines ophthalmochirurgischen Systems zur Behandlung eines Auges sowie ein ophthalmochirurgisches System zur Behandlung eines Auges gemäß den unabhängigen Ansprüchen vorgeschlagen.

Vorteilhafte Weiterbildungen ergeben sich durch Merkmale der abhängigen Ansprüche.

In Bezug auf eine gattungsgemäße Kassette für eine Konsole eines ophthalmochirurgischen Systems zur Behandlung eines Auges wird mit der Erfindung insbesondere vorgeschlagen, dass das Trennelement wenigstens ein Plattenelement aus einem zumindest elektrisch leitfähigen oder zumindest ferromagnetischen Werkstoff aufweist, welches Plattenelement bei einem Auslenken zumindest eines Bereichs des Trennelements mit ausgelenkt wird.

In Bezug auf eine gattungsgemäße Konsole eines ophthalmochirurgischen Systems zur Behandlung eines Auges wird mit der Erfindung insbesondere vorgeschlagen, dass die Sensoreinheit ausgebildet ist, unter Nutzung eines Magnetfeldes eine Auslenkungsposition des am Trennelement angeordneten Plattenelements einer Kassette gemäß der Erfindung zu erfassen.

In Bezug auf ein gattungsgemäßes ophthalmochirurgisches System zur Behandlung eines Auges wird mit der Erfindung insbesondere vorgeschlagen, dass zumindest die Kassette gemäß der Erfindung oder die Konsole gemäß der Erfindung ausgebildet ist.

Die Erfindung basiert unter anderem auf dem Gedanken, dass die Funktion der Regelung für das Behandlungsfluid verbessert werden kann, wenn die Eigenschaften der Fluidpumpe besser und genauer bekannt sind. Dies bezieht sich insbesondere auf die Auslenkungsposition des Trennelements. Da die Fluidpumpe in der Regel zum Fördern eines medizinischen Behandlungsfluids dient, sind häufig aus hygienischen Gründen und aus Gründen der Nachhaltigkeit keine Drucksensoren für das Behandlungsfluid vorgesehen. Eine Aussage über den Druck des Behandlungsfluids kann daher in einem solchen Fall nur indirekt erfolgen, das heißt, unter anderem abhängig vom Antriebsdruck des Antriebsfluids. Der tatsächliche Druck des Behandlungsfluids braucht dagegen nicht gemessen zu werden. Daher ist es wünschenswert, wenn die spezifische Auslenkungsposition des Trennelements möglichst genau erfasst und für das Steuern der Fluidpumpe berücksichtigt werden kann. Durch die erfindungsgemäße Konstruktion der Kassette, insbesondere in Verbindung mit der Konsole, kann erreicht werden, dass die Regelungsfunktion genauer und damit auch zielgerichteter realisiert werden kann.

Zu diesem Zweck wird die Auslenkungsposition des Trennelements mittels des Auslenkungspositionssensors der Konsole erfasst, wenn die Kassette in der Konsole angeordnet ist. Zu diesem Zweck weist das Trennelement das Plattenelement auf, welches beispielsweise als Metallplättchen ausgebildet sein kann. Das Plattenelement kann als flache, vorzugsweise ebene, Scheibe ausgebildet sein. Es kann einen umlaufenden Rand aufweisen, der zumindest teilweise rund oder eckig ausgebildet sein kann. Das Plattenelement kann aber auch eine vorgebbare Krümmung in der Ebene aufweisen, beispielsweise um eine Kontur des Trennelements, insbesondere im bestimmungsgemäßen Betrieb, unterstützen zu können. Das Plattenelement weist vorzugsweise zwei gegenüberliegende große Oberflächen auf, wobei einer der Oberflächen mit einer Oberfläche des Trennelements verbunden sein kann. Vorzugsweise ist das Plattenelement an einer Oberfläche des Trennelements angeordnet, welche der Antriebkammer zugewandt ist.

Dadurch dass das Trennelement zumindest einen elektrisch leitfähigen oder zumindest einen ferromagnetischen Werkstoff aufweist, ist es möglich, unter Nutzung eines magnetischen Feldes die Position des Plattenelements und damit die Auslenkungsposition des Trennelements zu erfassen. Hierfür weist die Konsole die Sensoreinheit auf, die vorzugsweise als induktive Sensoreinheit ausgebildet ist, ein geeignetes magnetisches Feld bereitstellt und Eigenschaften des magnetischen Feldes, die von der Position des Plattenelements abhängig sind, erfasst. Die Sensoreinheit kann abhängig vom erfassten magnetischen Feld ein Sensorsignal bereitstellen, welches einer Steuereinrichtung der Konsole zugeführt werden kann, um durch Auswerten des Sensorsignals die Auslenkungsposition des Trennelements zu bestimmen. Ist der Werkstoff des Plattenelements ferromagnetisch, kann vorgesehen sein, dass die Sensoreinheit ein im Wesentlichen konstantes magnetisches Feld für das Erfassen der Auslenkungsposition nutzt. Je nach Ausgestaltung kann jedoch auch ein magnetisches Wechselfeld vorgesehen sein. Ist der Werkstoff des Plattenelements dagegen lediglich elektrisch leitfähig, kann vorzugsweise nur ein magnetisches Wechselfeld vorgesehen sein. Natürlich können auch Kombinationen hiervon vorgesehen sein. Das Trennelement selbst ist insbesondere fluiddicht und ionendicht ausgebildet. Dadurch kann eine stoffdichte Trennung zwischen der Pumpkammer und der Antriebskammer erreicht werden.

Die Erfindung ermöglicht es daher, die Auslenkungsposition des Trennelements kontaktlos zu bestimmen. Dadurch dass die Sensoreinheit in der Konsole angeordnet ist und in der Kassette lediglich ergänzend das Plattenelement vorgesehen zu sein braucht, kann die Erfindung mit geringem Aufwand realisiert werden. Der Aufwand durch das Plattenelement ist für die in der Regel als austauschbares Einwegteil realisierte Kassette vergleichsweise gering. Die in der Konsole angeordnete Sensoreinheit kann dagegen mehrfach genutzt werden, sodass insgesamt auch hier der zusätzliche Aufwand gering gehalten werden kann. Die Kassette ist vorzugsweise sensoreinheitslos ausgebildet.

Das Trennelement, welches die Pumpkammer von der Antriebskammer trennt, kann als elastisches Trennelement nach Art einer elastischen Membran, einer Folie oder dergleichen ausgebildet sein. Darüber hinaus kann das Trennelement aber auch zumindest bereichsweise im Wesentlichen starr ausgebildet sein, wobei vorzugsweise der starre Bereich verlagerbar ist. Weiterhin kann das Trennelement beispielsweise einen Rand aufweisen, mittels dem es fest mit der Fluidpumpe verbunden sein kann. Das Trennelement kann zum Beispiel an seinem Rand in einer axialen Richtung, welche parallel zu einer Trennelement-Mittelachse verläuft, nicht verlagerbar sein. Insbesondere kann vorgesehen sein, dass das Trennelement an seinem Rand fest eingespannt ist. Vorzugsweise kann ein umfänglicher Rand des Trennelements fest in der Fluidpumpe angeordnet beziehungsweise befestigt sein. Dadurch kann das Trennelement die Pumpkammer von der Antriebskammer fluidtechnisch trennen, insbesondere derart, dass eine Sterilität des Behandlungsfluids nicht beeinträchtigt wird.

Die Fluidpumpe ist unter anderem vorzugsweise vollständig von der Kassette erfasst. Dies erlaubt es, die Kassette lösbar mit der Konsole zu verbinden, wobei im verbundenen Zustand die Fluidpumpe durch Anschließen der Antriebskammer an das Antriebsfluidversorgungssystem der Konsole zum Zuführen eines Antriebsfluids zu der Antriebskammer fluidtechnisch gekoppelt werden kann. Im mit der Konsole verbundenen Zustand der Kassette kann die Sensoreinheit im Bereich der Fluidpumpe der Kassette positioniert sein, sodass eine zuverlässige Funktion gewährleistet werden kann.

Dadurch kann mit der Kassette ein Austauschteil beziehungsweise Einwegteil bereitgestellt werden, welches dazu dienen kann, eine Sterilität des ophthalmochirurgischen Systems für eine jeweilige Operation am Auge, insbesondere in Bezug auf das Behandlungsfluid, sicherstellen zu können. Es ist dadurch nämlich möglich, dass Behandlungsfluid nur durch die Kassette geführt zu werden braucht, sodass das Behandlungsfluid die Konsole nicht zu durchströmen und auch nicht mit der Konsole in Kontakt zu kommen braucht. Dadurch kann nach einer jeweiligen Nutzung des ophthalmochirurgischen Systems durch Austauschen der Kassette in der Konsole die Sterilität des ophthalmochirurgischen Systems jederzeit auf einfache Weise wiederhergestellt werden.

Natürlich ist im mit der Konsole verbunden Zustand vorgesehen, dass die Kassette fluiddicht mit der Konsole verbunden ist, sodass die jeweilige Antriebskammer der jeweiligen Fluidpumpe fluiddicht mit dem Antriebsfluidversorgungssystem gekoppelt werden kann, um die Fluidpumpe bestimmungsgemäß betreiben zu können. Beispielsweise kann vorgesehen sein, dass die Kassette, insbesondere auch die Fluidpumpe beziehungsweise die kassettenseitigen Elemente der Fluidpumpe, insbesondere das jeweilige Trennelement, aus einem geeigneten Kunststoff oder vergleichbarem Material hergestellt sein können.

Das Antriebsfluid, welches der Antriebskammer zuführbar ist, um die Fluidpumpe antreiben zu können, kann zum Beispiel eine Flüssigkeit wie beispielsweise Wasser, Öl, Mischungen von Flüssigkeiten und/oder dergleichen, sowie auch ein Gas, beispielsweise Luft, Stickstoff, ein Edelgas, Mischungen von Gasen und/oder dergleichen, sowie eine Kombination hiervon sein.

Das Antriebsfluid kann vorzugsweise ausschließlich mittels der Konsole bereitgestellt werden. In der Konsole kann zu diesem Zweck das Antriebsfluidversorgungssystem zum Beispiel einen Antriebsdrucksensor aufweisen, mittels welchem der Antriebsdruck des Antriebsfluids erfasst werden kann. Dadurch, dass der Antriebsdrucksensor konsolenseitig vorgesehen sein kann, braucht er, weil er mit dem Behandlungsfluid nicht in Kontakt zu kommen braucht, hinsichtlich der Sterilität keine besonderen Anforderungen zu erfüllen. Dadurch kann der Antriebsdrucksensor bezüglich seiner Erfassungsfunktionalität optimiert ausgewählt sein.

Ferner ist konsolenseitig die Sensoreinheit vorgesehen, die dazu dient, die Auslenkungsposition des Trennelements zu erfassen. Die Sensoreinheit kann die Auslenkungsstellung des Trennelements vorzugsweise kontaktlos erfassen. Hierzu kann die Sensoreinheit zum Beispiel ein induktives Sensorelement aufweisen. Die Sensoreinheit kann leitungsgebunden oder drahtlos, beispielsweise über Funk oder auch über eine Kommunikationsleitung, mit einer Steuereinrichtung des ophthalmochirurgischen Systems, insbesondere der Konsole, kommunizieren. Die Sensoreinheit kann zum Beispiel als Transponder, insbesondere als passiver Transponder, ausgebildet sein.

Das Plattenelement kann eine runde und/oder eine eckige Kontur aufweisen. Es ist vorzugsweise im Wesentlichen eben beziehungsweise flach ausgebildet. In alternativen Ausgestaltungen kann das Plattenelement jedoch auch zumindest teilweise gekrümmt ausgebildet sein. Das Plattenelement ist vorzugsweise im Wesentlichen starr ausgebildet, sodass es sich im bestimmungsgemäßen Betrieb kaum oder im Wesentlichen nicht verformt. Dadurch kann das Plattenelement auf dem Trennelement eine zusätzliche Stabilität hinsichtlich der Verformung geben. Das Plattenelement kann auch mehrteilig ausgebildet sein, beispielsweise einen Schichtaufbau aufweisen oder dergleichen. Darüber hinaus kann das Plattenelement zumindest teilweise aus einem federelastischen Werkstoff gebildet sein.

Die Kassette kann ein Kassettengehäuse aufweisen, welches aus einem geeigneten Werkstoff, beispielsweise einem Kunststoff oder dergleichen, gebildet sein kann. Die Fluidpumpe kann zumindest teilweise einstückig mit dem Gehäuse ausgebildet sein.

Es wird weiterhin vorgeschlagen, dass das Plattenelement eine Dicke aufweist, die kleiner als eine Dicke des Trennelements ist. Dadurch kann das Plattenelement zum Beispiel auch in das Trennelement integriert angeordnet sein. Beispielsweise kann das Plattenelement von einem Werkstoff des Trennelements umgeben sein, indem es zum Beispiel umspritzt wird.

Darüber hinaus ist das Plattenelement von einer Einfassung beabstandet angeordnet, welche das Trennelement hält. Die Einfassung kann von einem Gehäuse der Kassette bereitgestellt sein, und zum Beispiel eine Nut aufweisen, in der ein Rand des Trennelements befestigt ist. Die Einfassung ist vorzugsweise derart ausgestaltet, dass eine fluiddichte Trennung zwischen der Antriebskammer und der Pumpkammer realisiert werden kann. Damit die fluiddichte Trennung möglichst nicht beeinträchtigt wird, ist das Plattenelement beabstandet von der Einfassung angeordnet. Die Einfassung kann eine stoffschlüssige Verbindung eines Gehäuses der Kassette mit dem Trennelement realisieren. Darüber hinaus kann natürlich auch vorgesehen sein, dass die Einfassung Dichtungsmittel umfasst, die neben einer fluidtechnischen Abdichtung auch eine Haltefunktion für das Trennelement bereitzustellen vermögen. Es kann auch eine Pressverbindung vorgesehen sein. Darüber hinaus kann natürlich auch vorgesehen sein, dass das Trennelement zusammen mit der Einfassung und dem Gehäuse der Kassette einstückig ausgebildet ist.

Das Trennelement weist antriebskammerseitig einen, insbesondere zentralen, Aufnahmebereich zum Anordnen des Plattenelements auf. Durch den Aufnahmebereich kann erreicht werden, dass das Plattenelement als separates Teil mit dem Trennelement verbunden werden kann. Das Trennelement und das Plattenelement können somit separat voneinander hergestellt werden. Der Aufnahmebereich kann zum Beispiel durch eine Tasche gebildet sein, die das Trennelement, insbesondere antriebskammerseitig, bereitstellt. In die Aufnahmetasche kann das Plattenelement bei der Herstellung der Kassette eingesetzt werden. Der Aufnahmebereich ist vorzugsweise zum Aufnehmen des Plattenelements angepasst ausgebildet. Besonders vorteilhaft erweist es sich, wenn der Aufnahmebereich zentral in Bezug auf eine Erstreckung des Trennelements angeordnet ist. Dadurch kann das Plattenelement beispielsweise im Wesentlichen mittig mit Bezug auf das Trennelement angeordnet sein, wodurch die Funktion der Fluidpumpe unterstützt werden kann.

Darüber hinaus läuft ein Ringbereich in Umfangsrichtung um den Aufnahmebereich des Trennelements um. Dadurch kann erreicht werden, dass zumindest ein Bereich des Trennelements in Umfangsrichtung, beispielsweise im Bereich der Einfassung, elastisch bewegbar bleibt, um die Pumpfunktion zuverlässig realisieren zu können. Zugleich kann erreicht werden, insbesondere wenn der Aufnahmebereich zentral angeordnet ist, dass in einem mittleren Bereich des Trennelements eine reduzierte Elastizität durch das Plattenelement realisiert werden kann, wodurch die Auslenkungsposition besser definiert und mittels der Sensoreinheit besser erfasst werden kann. Vorzugsweise weist der Ringbereich in Umfangsrichtung einen im Wesentlichen konstanten Radius auf. Das Trennelement ist tellerförmig ausgebildet.

Das Plattenelement kann vorzugsweise arbeitskammerseitig an einer Oberfläche des Trennelements angeordnet, insbesondere befestigt, sein. Die Befestigung des Plattenelements an der Oberfläche des Trennelements kann beispielsweise mittels einer Klebverbindung, einer Schweißverbindung oder dergleichen realisiert sein. Die geringe Dicke des Plattenelements ermöglicht darüber hinaus, dass das Plattenelement ein geringes Gewicht aufweist, sodass die Funktion der Fluidpumpe, insbesondere des Trennelements im bestimmungsgemäßen Betrieb der Fluidpumpe möglichst wenig, vorzugsweise im Wesentlichen nicht, beeinflusst wird.

Es wird ferner vorgeschlagen, dass das Plattenelement eine Beschichtung aufweist. Die Beschichtung kann dazu dienen, das Plattenelement vor Wechselwirkungen mit dem Trennelement und/oder dem Antriebsfluid zu schützen. Die Beschichtung kann daher beispielsweise eine passivierende Wirkung vorsehen. Die Beschichtung kann beispielsweise durch ein Harz, einen Kunststoff oder dergleichen gebildet sein, die auf einer Oberfläche des Plattenelements oder allen Oberflächen des Plattenelements aufgebracht ist. Die Beschichtung kann dem Grunde nach jedoch auch eine chemische Schicht sein, die eine passivierende Wirkung realisiert, so beispielsweise bei einem Plattenelement aus Aluminium in Verbindung mit einem Arbeitsfluid aus Luft, wobei sich als Beschichtung eine Aluminiumoxidschicht an der Oberfläche des Plattenelements ausbilden kann.

Weiterhin wird vorgeschlagen, dass das Trennelement antriebskammerseitig wenigstens ein Verbindungselement zum mechanischen Verbinden des im Aufnahmebereich angeordneten Plattenelements mit dem Trennelement aufweist. Das Verbindungselement ermöglicht es vorzugsweise, das Plattenelement lösbar mit dem Trennelement zu verbinden. Dies ist besonders vorteilhaft, wenn die Kassette als Einwegteil oder Wegwerfteil realisiert ist und eine Werkstofftrennung zum Zwecke des Recycelns vorgesehen werden soll. Darüber hinaus kann durch das Verbindungselement eine einfache Montage des Plattenelements am Trennelement erreicht werden. Beispielsweise kann das Verbindungselement das Plattenelement an vorgebbaren Positionen mit dem Trennelement mechanisch verbinden. Es kann aber auch als ein einziges Teil vorgesehen sein, welches das Plattenelement hält.

Ferner wird vorgeschlagen, dass das wenigstens eine Verbindungselement wenigstens eine Aufnahmenut für einen Rand des Plattenelements aufweist. Dadurch kann erreicht werden, dass das Plattenelement durch Einschieben oder Einclipsen des Randes in die Aufnahmenut mit dem Trennelement verbunden werden kann. Dies ermöglicht eine einfache Montage, die zugleich auch die Realisierung einer ausreichenden Sterilität ermöglicht.

Das ophthalmochirurgische System zur Behandlung eines Auges weist zumindest eine Konsole zur Aufnahme eines Behandlungsfluidbehälters zum Aufnehmen eines Behandlungsfluids auf. Der Behandlungsfluidbehälter kann zum Beispiel ein Irrigationsfluidbehälter sein, der ein ausreichend großes Fassungsvermögen für ein Irrigationsfluid hat, sodass auch eine längere Operation ohne Wechsel des Behandlungsfluidbehälters durchgeführt werden kann. Das Gleiche kann dem Grunde nach auch für einen Behandlungsfluidbehälter vorgesehen sein, der zum Aufnehmen des Aspirationsfluids dient. Das ophthalmochirurgische System weist ferner die in die Konsole einsetzbare Kassette zur Steuerung des Behandlungsfluids zum chirurgischen Instrument zur Behandlung des Auges auf. Die Kassette ist vorzugsweise derart ausgebildet, dass das Behandlungsfluid mit der Konsole nicht in Kontakt kommt. Die Kassette kann als Einwegbauteil ausgebildet sein, sodass es nach einer Nutzung bei einer Operation an einem Auge für eine darauffolgende Operation ausgetauscht werden kann. Dadurch kann auf einfache Weise eine Sterilität hergestellt werden. Das ophthalmochirurgische System weist ferner wenigstens eine Fluidpumpe zum Fördern des Behandlungsfluids im bestimmungsgemäßen Betrieb des Systems auf, die vorzugsweise in der Kassette angeordnet ist. Vorzugsweise ist wenigstens eine Fluidpumpe zum Fördern des Irrigationsfluids und wenigstens eine Pumpe zum Fördern des Aspirationsfluids vorgesehen. Die jeweilige Fluidpumpe weist eine Pumpkammer und eine mittels des Trennelements von der Pumpkammer getrennte Antriebskammer auf. Die Antriebskammer ist mit dem Antriebsfluid beaufschlagbar, das konsolenseitig der Antriebskammer zugeführt werden kann. Zu diesem Zweck kann eine Antriebsfluidquelle vorgesehen sein, die hinsichtlich des Antriebsdrucks einstellbar ausgebildet ist. Der Antriebsdruck kann mittels eines Antriebsdrucksensors erfasst werden, der ebenfalls konsolenseitig angeordnet sein kann. Durch diese Ausgestaltung der Fluidpumpe ist es möglich, dass das Behandlungsfluid in der Kassette im Wesentlichen getrennt von der Konsole geführt werden kann. Lediglich Anschlüsse für ein Zuführen des Behandlungsfluids zur Fluidpumpe und für ein Abführen des Behandlungsfluids von der Fluidpumpe brauchen an der Kassette vorgesehen zu sein.

Das ophthalmochirurgische System umfasst ferner eine Sensoreinheit zum Erfassen der Auslenkungsposition des Trennelements. Die Sensoreinheit ist vorzugsweise konsolenseitig angeordnet. Der Sensoreinheit kann vorzugsweise drahtlos kommunikationstechnisch mit der Steuereinrichtung, insbesondere der vorzugsweise in der Konsole angeordneten Steuereinrichtung des ophthalmochirurgischen Systems, gekoppelt sein.

Die Konsole kann darüber hinaus die Steuereinrichtung aufweisen, die die erforderlichen Funktionen für den bestimmungsgemäßen Betrieb sowie auch insbesondere für die erfindungsgemäßen Verfahrensführungen realisieren kann. Zu diesem Zweck kann die Steuereinrichtung an die entsprechenden Sensoren und Antriebs- beziehungsweise Steuerelemente angeschlossen sein.

Die für die erfindungsgemäße Kassette sowie die erfindungsgemäße Konsole angegebenen Vorteile und Wirkungen gelten natürlich gleichermaßen auch für das erfindungsgemäße ophthalmochirurgische System und umgekehrt.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Fig. nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen.

In den Figuren zeigen:
- Fig. 1: Eine schematische Darstellung einer ersten Ausführungsform des erfindungsgemäßen ophthalmochirurgischen Systems,
- Fig. 2: eine schematisch perspektivische Darstellung einer Konsole des Systems gemäß Fig. 1,
- Fig. 3: eine schematische Draufsicht auf eine Anschlussseite einer Kassette für das System gemäß Fig. 1, und
- Fig. 4: eine schematische Schnittdarstellung eines Ausschnitts der in einem Kassettenaufnahmebereich der Konsole gemäß Fig. 2 angeordneten Kassette gemäß Fig. 3.

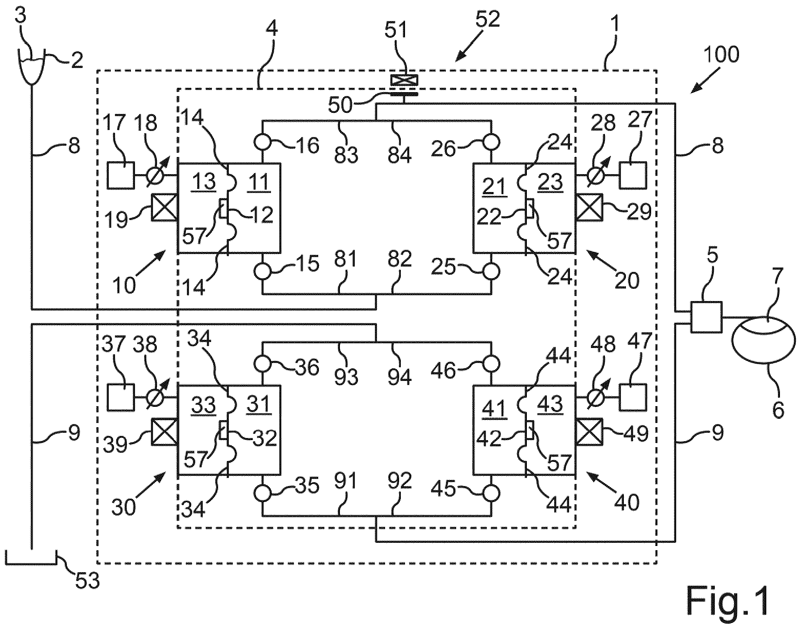

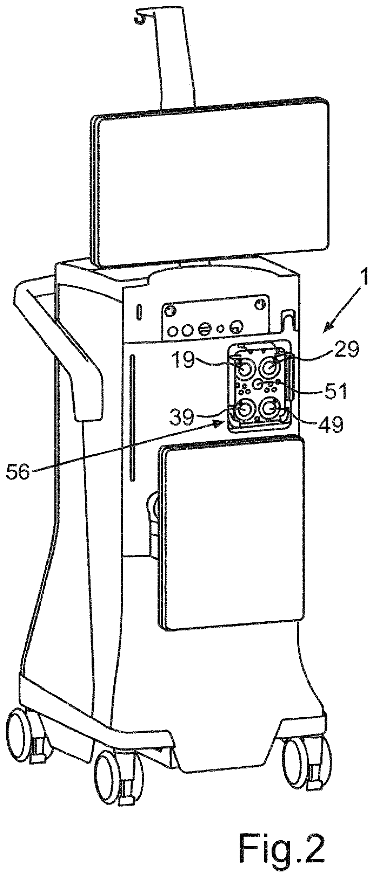

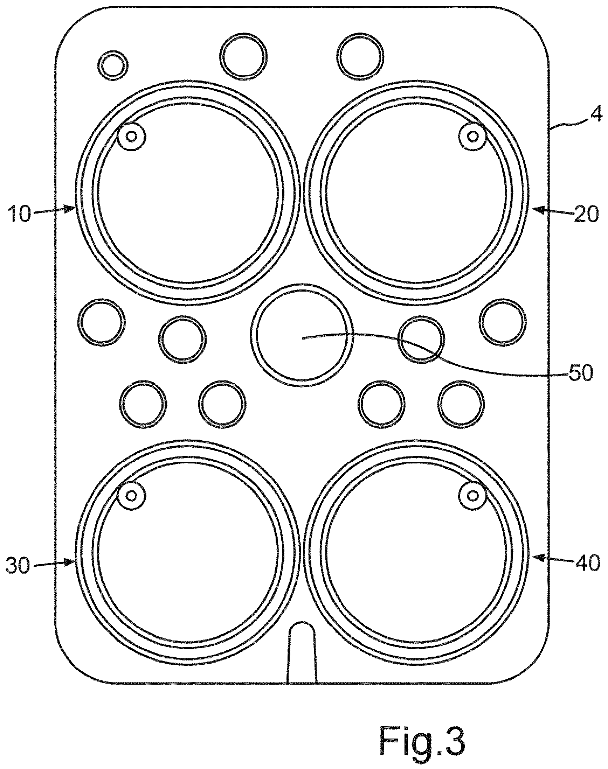

Fig. 1 zeigt eine schematische Darstellung einer ersten Ausführungsform eines erfindungsgemäßen ophthalmochirurgischen Systems 100. Das System 100 weist eine Konsole 1 auf, an welcher ein Irrigationsfluidbehälter 2 mit einem darin enthaltenen Irrigationsfluid 3 gekoppelt ist. Weiterhin weist das System 100 eine Kassette 4 auf, welche in die Konsole 1 einsetzbar ist. Die Kassette 4 dient neben einer Förderung des Irrigationsfluids 3 zu einem chirurgischen Instrument 5 als medizinisches Behandlungsinstrument, welches zur Phakoemulsifikation einer Linse 7 eines Auges 6 als Behandlung dient, auch zum Abführen eines hierbei anfallenden Aspirationsfluids aus einem Behandlungsbereich des Auges 6. Das chirurgische Instrument 5 ist vorliegend als Handstück ausgebildet und dient zur Phakoemulsifikation der Linse 7 des Auges 6. Fig. 2 zeigt in einer schematischen perspektivischen Darstellung die Konsole 1 ohne die Kassette 4. Fig. 3 zeigt in einer schematischen Draufsicht eine Anschlussseite der Kassette 4 zum Verbinden mit der Konsole 1.

Das System 100 weist ferner einen Irrigationsfluid-Strömungspfad 8 auf, der sich vom Irrigationsfluidbehälter 2 über die Kassette 4 zum chirurgischen Instrument 5 erstreckt. Darüber hinaus weist das System 100 eine erste Fluidpumpe 10 mit einer ersten Pumpkammer 11 und eine mit einem ersten Trennelement 12 davon getrennten ersten Antriebskammer 13 auf. Das Trennelement 12 ist bevorzugt verformbar ausgebildet. Das erste Trennelement 12 weist einen Rand 14 auf, mittels dem es fest in der Fluidpumpe 10 verbunden ist. Das Trennelement 12 ist an seinem Rand 14 in einer axialen Richtung, welche parallel zu einer Trennelement-Mittelachse verläuft, nicht verlagerbar. Bevorzugt ist das Trennelement 12 an seinem Rand 14 fest eingespannt.

Das Irrigationsfluid 3 ist über den Irrigationsfluid-Strömungspfad 8 und einem ersten Einlassventil 15 der ersten Pumpkammer 11 abhängig von einem Ventilzustand des Einlassventils 15 der ersten Pumpkammer 11 zuführbar. Ferner ist es über ein Auslassventil 16 abhängig von dessen Ventilzustand wieder von der Pumpkammer 11 abführbar. Die erste Antriebskammer 13 kann mit einem ersten Antriebsfluid 17 beaufschlagt werden, welches mittels eines in der Konsole 1 angeordneten Proportionalventils 18 zugeführt werden kann. Abhängig von einem Differenzdruck zwischen dem ersten Antriebsfluid 17 in der ersten Antriebskammer 13 und dem Irrigationsfluid 3 als Behandlungsfluid in der ersten Pumpkammer 11 kommt es zu einer bereichsweisen Auslenkung des ersten Trennelements 12. Dabei ist ein Betrag des Drucks in der ersten Antriebskammer 13 größer als ein Betrag des Drucks in der ersten Pumpkammer 11. Wenn das Einlassventil 15 geschlossen und das Auslassventil 16 geöffnet ist, kann das Irrigationsfluid 3 aus der ersten Pumpkammer 11 in einen an das Auslassventil angeschlossenen Teilpfad 83 ausströmen.

Eine Auslenkungsposition des ersten Trennelements 12 kann mittels eines ersten Auslenkungspositionssensors 19 erfasst werden, welcher außerhalb der ersten Fluidpumpe 10 zum Beispiel in der Konsole 1 angeordnet ist. Der erste Auslenkungspositionssensor 19 ist vorliegend als Sensoreinheit nach Art eines induktiven Wegsensors ausgebildet. Die Funktion des Auslenkungspositionssensors 19 wird im Folgenden noch erläutert werden.

Wie aus Fig. 1 ersichtlich ist, sind die Antriebskammer 13 und die Pumpkammer 11 mit dem Trennelement 12 in der Kassette 4 angeordnet. Dadurch wird durch Anordnen der Kassette 4 in der Konsole 1 die Fluidpumpe 10 mit einer Antriebsfluidzuführung der Konsole 1 und dem in der Konsole 1 angeordneten Auslenkungspositionssensor 19 gekoppelt, sodass die gewünschte Pumpfunktion und die Erfassungsfunktion für das Erfassen der Auslenkungsposition des ersten Trennelements 12 erreicht werden kann.

Aus Fig. 1 ist ferner ersichtlich, dass zur Fluidpumpe 10 eine zweite Fluidpumpe 20 strömungstechnisch parallelgeschaltet ist. Die Fluidpumpe 20 ist vorliegend wie die Fluidpumpe 10 ausgebildet. Daher teilt sich der Irrigationsfluid-Strömungspfad 8 in der Kassette 4 in einen ersten Teilpfad 81 und einen zweiten Teilpfad 82 auf. Der erste Teilpfad 81 ist an das erste Einlassventil 15 und der zweite Teilpfad 82 an ein zweites Einlassventil 25 der zweiten Fluidpumpe 20 angeschlossen.

Die zweite Fluidpumpe 20 weist eine zweite Pumpkammer 21 und eine hiervon mittels eines zweiten Trennelements 22 getrennte zweite Antriebskammer 23 auf. Das Trennelement 22 weist einen zweiten Rand 24 auf, der fest in der zweiten Fluidpumpe 20 montiert ist. Die zweite Antriebskammer 23 kann mit einem zweiten Antriebsfluid 27 über ein in der Konsole 1 angeordnetes zweites Proportionalventil 28 beaufschlagt werden. Eine Auslenkungsposition des Trennelements 22 kann mittels eines Auslenkungspositionssensors 29 erfasst werden, der vorliegend entsprechend dem Auslenkungspositionssensor 19 ausgebildet ist. Über ein zweites Auslassventil 26 kann das Irrigationsfluid 3 die zweite Pumpkammer 21 in den Teilpfad 84 wieder verlassen. Über die Teilpfade 83, 84, die am jeweiligen ersten beziehungsweise zweiten Auslassventil 16, 26 angeschlossen sind, kann das die jeweilige Fluidpumpe 10, 20 verlassende Irrigationsfluid wieder dem Irrigationsfluid-Strömungspfad 8 zugeführt werden, um dem Instrument 5 zugeführt zu werden.

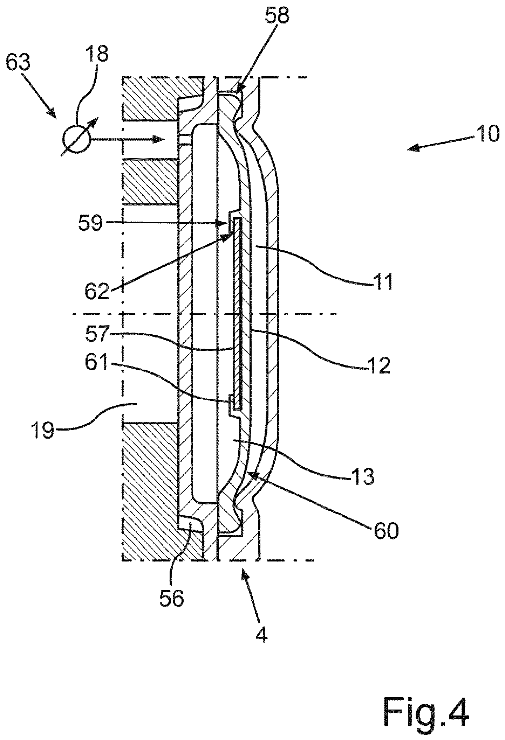

In einem Bereich der strömungstechnischen Verbindung des Teilpfades 83 mit dem Teilpfad 84, also zum Beispiel in dem nachfolgenden Irrigationsfluid-Strömungspfad 8, ist eine elastische Membran 50 ausgebildet, die das Irrigationsfluid 3 kontaktieren kann. Die Membran 50 ist an der Kassette 4 angeordnet. Die Membran 50 wird von einem Kraftsensor 51 kontaktiert, der seinerseits in der Konsole 1 angeordnet ist, wenn die Kassette 4 im Kassettenaufnahmebereich 56 angeordnet ist (Fig. 4). Die Membran 50 bildet in Verbindung mit dem Kraftsensor 51 einen Erfassungssensor 52.

Während des Zerkleinerns der Augenlinse 7 werden kleine Linsenpartikel freigesetzt, die zusammen mit dem zugeführten Irrigationsfluid abgesaugt werden können. Das mit Linsenpartikeln verunreinigte Irrigationsfluid wird dann als Aspirationsfluid bezeichnet und über einen Aspirationsfluid-Strömungspfad 9 zu einem Aspirationsfluid-Sammelbehälter 53 gefördert. Zu diesem Zweck sind vorliegend zwei weitere parallelgeschaltete Fluidpumpen 30, 40 vorgesehen, die dem Grunde nach vergleichbar zu den Fluidpumpen 10, 20 für das Irrigationsfluid ausgebildet sind. Zu diesem Zweck ist innerhalb der Kassette 4 vorgesehen, dass der Aspirations-Strömungspfad 9 sich ebenfalls in zwei Teilpfade 91, 92 aufteilt, die über jeweilige Einlassventile 35, 45 an die jeweiligen Fluidpumpen 30, 40 angeschlossen sind, und zwar hier zu den jeweiligen Pumpkammern 31, 41. Auch hier sind die Pumpkammern 31, 41 über jeweilige Trennelemente 32, 42 von jeweiligen Antriebskammern 33, 43 getrennt. Die Trennelemente 32, 42 weisen jeweilige Ränder 34, 44 auf, die fest in der jeweiligen Fluidpumpe 30, 40 montiert sind. Über jeweilige Auslassventile 36, 46 und daran angeschlossene Teilpfade 93, 94 kann das Aspirationsfluid dann über den Aspirationsfluid-Strömungspfad 9 abgeführt werden. Ein drittes Antriebsfluid 37 kann mittels eines dritten Proportionalventils 38 zur dritten Antriebskammer 33 geführt werden. Entsprechend kann ein viertes Antriebsfluid 47 mittels eines vierten Proportionalventils 48 zu einer vierten Antriebskammer 43 geführt werden. Die Proportionalventile 38, 48 sind in der Konsole 1 angeordnet. Die Auslenkungspositionen der Trennelemente 32, 42 können mittels jeweiliger Auslenkungspositionssensoren 39, 49 erfasst werden. Die beiden Fluidpumpen 30, 40 werden vorliegend ebenfalls wechselweise wie die Fluidpumpen 10, 20 betrieben.

Fig. 2 zeigt in einer schematisch perspektivischen Darstellung die Konsole 1 des ophthalmochirurgischen Systems 100 gemäß Fig. 1. Aus Fig. 2 ist ersichtlich, dass die Konsole 1 den Kassettenaufnahmebereich 56 aufweist, der dem lösbaren Anordnen der Kassette 4 dient. Die Konsole 1 weist ferner ein Antriebsfluidversorgungssystem 63 auf, welches dazu dient, das jeweilige Antriebsfluid für die jeweiligen Antriebskammern 13, 23, 33, 43 bereitzustellen, welches vorliegend durch Luft gebildet ist. Das Antriebsfluidversorgungssystem 63 weist für jede der Fluidpumpen 10, 20, 30, 40 jeweilige Antriebsfluidquellen 17, 27, 37, 47 (Fig. 1) auf, die an jeweilige Proportionalventile 18, 28, 38, 48 angeschlossen sind. Über die Proportionalventile 18, 28, 38, 48 kann die jeweilige Antriebskammer 13, 23, 33, 43 im bestimmungsgemäßen Betrieb mit dem Antriebsfluid beaufschlagt werden.

Aus Fig. 1 ist ferner ersichtlich, dass jedes der Trennelemente 12, 22, 32, 42 ein Plattenelement 57 aufweist, welches bei einem Auslenken eines zentralen Bereichs des Trennelements 12, 22, 32, 42 mit ausgelenkt wird. Das Plattenelement 57 ist vorliegend als Metallplättchen ausgebildet und aus einem ferromagnetischen Werkstoff gebildet. Das Plattenelement 57 weist eine Dicke auf, die kleiner als eine Dicke des Trennelements 12, 22, 32, 42 ist.

Die Konsole 1 weist ferner für jede der Fluidpumpen 10, 20, 30, 40 einen jeweiligen Auslenkungspositionssensor 19, 29, 39, 49 auf, der vorliegend jeweils eine Sensoreinheit bereitstellt, die ein magnetisches Feld nutzt, um die Position des jeweiligen Plattenelements 57 und damit des jeweiligen der Trennelemente 12, 22, 32, 42 zu erfassen.

Fig. 4 zeigt schematisch einen Ausschnitt der in der Kassettenaufnahme 56 der Konsole 1 gemäß Fig. 2 angeordneten Kassette 4 gemäß Fig. 3, und zwar im Bereich der Fluidpumpe 10. Die folgenden Ausführungen gelten gleichermaßen für die Fluidpumpen 20, 30, 40. Aus Fig. 4 ist ersichtlich, dass das Plattenelement 57 von einer Einfassung 58 eines nicht weiter bezeichneten Gehäuses der Kassette 4 beabstandet angeordnet ist, welche das Trennelement 12 hält.

Das Plattenelement 57 ist am Trennelement 12 antriebskammerseitig vorgesehen, sodass die Pumpkammer 11 durch das Trennelement 12 vom Plattenelement 57 getrennt angeordnet ist. Dies ist vorteilhaft aus Gründen der Sterilität. Um das Plattenelement 57 vor Korrosion aufgrund einer Wechselwirkung mit dem Antriebsfluid zu schützen, weist das Plattenelement 57 eine Beschichtung auf, die vorliegend durch ein Harz gebildet ist.

Das Trennelement 12 weist antriebskammerseitig einen zentralen Aufnahmebereich 59 zum Anordnen des Plattenelements 57 auf, wobei ein Ringbereich 60 des Trennelements 12 in Umfangsrichtung um den Aufnahmebereich 59 umläuft. Vorliegend ist der Aufnahmebereich 59 im zentralen Bereich des Trennelements 12 positioniert. Dadurch kann sichergestellt werden, dass das Trennelement 12 für eine bestimmungsgemäße Pumpfunktion im Wesentlichen unbeeinträchtigt bewegt werden kann.

Um das Trennelement 12 mit dem Plattenelement 57 zu verbinden, ist vorliegend vorgesehen, dass das Trennelement 12 antriebskammerseitig mit einem Verbindungselement 61 zum mechanischen Verbinden des im Aufnahmebereich 59 angeordneten Plattenelements 57 mit dem Trennelement 12 versehen ist. Das Verbindungselement 61 ist vorliegend nach Art eines Rahmens ausgebildet, der eine Aufnahmenut 62 für einen Rand des Plattenelements 57 aufweist. Insbesondere ist es auf diese Weise möglich, nach Beendigung der Nutzung der Kassette 4 das jeweilige Plattenelement 57 auf einfache Weise von dem jeweiligen Trennelement 12, 22, 32, 42 zu entfernen, um die Stoffe getrennt weiter verwerten zu können.

Dem Grunde nach kann jedoch auch eine stoffschlüssige Verbindung vorgesehen sein, in dem das Plattenelement 57 von einem Werkstoff des Trennelements 12 vollständig umgeben ist. Dies kann zum Beispiel während der Herstellung des Trennelements 12 erfolgen, indem das Plattenelement 57 vom Werkstoff umspritzt wird oder dergleichen. Dem Grunde nach kann auch eine Klebeverbindung zwischen dem Trennelement 12 und dem Plattenelement 57 vorgesehen sein. Diese Überlegungen gelten natürlich gleichermaßen auch für die weiteren Fluidpumpen 20, 30, 40.

Das Trennelement 12 ist an einem Gehäuse der Kassette 4 in einer Einfassung 58 gehalten. Die Einfassung 58 dichtet nicht nur die Pumpkammer 11 von der Antriebskammer 13 ab, sondern sie sorgt auch für eine zuverlässige Positionierung des Trennelements 12 während des bestimmungsgemäßen Betriebs der Fluidpumpe 10.

Der Auslenkungspositionssensor 19 ist vorliegend als induktiver Sensor ausgebildet, der beispielsweise unter Nutzung eines im Wesentlichen konstanten Magnetfelds die Position des Plattenelements 57 erfasst und ein entsprechendes Sensorsignal an eine nicht dargestellte Steuereinrichtung der Konsole 1 übermittelt. Die Steuereinrichtung der Konsole 1 kann dieses Signal auswerten und die Fluidpumpe 10 sowie die zugehörigen Ventile entsprechend steuern.

Das Trennelement 12 weist in dieser Ausgestaltung eine Geometrie nach Art einer kreisförmigen Scheibe auf. Bei alternativen Ausgestaltungen kann jedoch auch eine zumindest teilweise eckige Kontur vorgesehen sein. So kann das Plattenelement auch rechteckig oder dergleichen ausgebildet sein.

Das Trennelement 12 weist in dieser Ausgestaltung ferner eine Wölbung in einem Randbereich, welcher der Ringbereich 60 ist, auf. Das Trennelement 12 verläuft von seiner Einfassung 58 ausgehend im Querschnitt somit nicht plan weiter, sondern gewölbt, sodass das Trennelement 12 im Querschnitt tellerförmig ausgebildet ist. Dieser tellerförmig ausgewölbte Randbereich 60 ist vorteilhaft, da sich das Trennelement 12 bei Zufuhr von Antriebsfluid in die Antriebskammer 13 in Richtung zu einer Innenwand der Fluidkammer 11 auswölben und sich an diese Innenwand vollflächig und homogen anlegen kann. Damit kann das in der Pumpkammer 11 enthaltene Behandlungsfluid vollständig abgeführt werden und die Pumpkammer vollständig entleert werden. Somit verbleibt kein Rest an Behandlungsfluid in der Pumpkammer 11, sodass ein maximal großes Fördervolumen zugeführt und abgeführt werden kann. Zusätzlich wird durch den tellerförmig gewölbten Randbereich 60 erreicht, dass das Plattenelement 57 mit hoher Genauigkeit entlang der Mittelachse des Trennelementes 12 bewegbar ist und keine Taumelbewegung des Plattenelementes 57 auftritt. Das Trennelement 12 ist bevorzugt so dimensioniert, dass eine Federkonstante von weniger als 50mmHg/10mm erreicht wird.

Die Ausführungsbeispiele dienen ausschließlich der Erläuterung der Erfindung und sollen diese nicht beschränken.

### Bezugszeichenliste

- 1: Konsole
- 2: Irrigationsfluidbehälter
- 3: Irrigationsfluid
- 4: Kassette
- 5: chirurgisches Instrument
- 6: Auge
- 7: Augenlinse
- 8: Irrigationsfluid-Strömungspfad
- 9: Aspirations-Strömungspfad
- 10: Fluidpumpe
- 11: Pumpkammer
- 12: Trennelement
- 13: Antriebskammer
- 14: Rand
- 15: Einlassventil
- 16: Auslassventil
- 17: Antriebsfluidquelle
- 18: Proportionalventil
- 19: Auslenkungspositionssensor
- 20: Fluidpumpe
- 21: Pumpkammer
- 22: Trennelement
- 23: Antriebskammer
- 24: Rand
- 25: Einlassventil
- 26: Auslassventil
- 27: Antriebsfluidquelle
- 28: Proportionalventil
- 29: Auslenkungspositionssensor
- 30: Fluidpumpe
- 31: Pumpkammer
- 32: Trennelement
- 33: Antriebskammer
- 34: Rand
- 35: Einlassventil
- 36: Auslassventil
- 37: Antriebsfluidquelle
- 38: Proportionalventils
- 39: Auslenkungspositionssensor
- 41: Pumpkammer
- 42: Trennelement
- 43: Antriebskammer
- 44: Rand
- 45: Einlassventil
- 46: Auslassventil
- 47: Antriebsfluidquelle
- 48: Proportionalventil
- 49: Auslenkungspositionssensor
- 50: Membran
- 51: Kraftsensor
- 52: Erfassungssensor
- 53: Aspirationsfluid-Sammelbehälter
- 54: Verstelleinrichtung
- 55: Kopplung
- 56: Kassettenaufnahmebereich
- 57: Metallplättchen
- 58: Einfassung
- 59: Aufnahmebereich
- 60: Ringbereich
- 61: Verbindungselement
- 62: Nut
- 63: Antriebsfluidversorgungssystem
- 81, 82, 83, 84, 91, 92, 93, 94: Teilpfad
- 100: ophthalmochirurgisches System

## Patentansprüche

1. Kassette (4) für eine Konsole (1) eines ophthalmochirurgischen Systems (100) zur Behandlung eines Auges (6), wobei die Kassette (4) zum Einsetzen in einen Kassettenaufnahmebereich (56) der Konsole (1) ausgebildet ist und wenigstens eine Fluidpumpe (10, 20, 30, 40) zum Fördern eines Behandlungsfluids aufweist, wobei die Fluidpumpe (10, 20, 30, 40) eine Pumpkammer (11, 21, 31, 41) und eine mittels eines zumindest bereichsweise auslenkbaren fluiddichten und ionendichten Trennelements (12, 22, 32, 42) von der Pumpkammer (11, 21, 31, 41) getrennte Antriebskammer (13, 23, 33, 43) aufweist, wobei der Antriebskammer (13, 23, 33, 43) ein Antriebsfluid und der Pumpkammer das Behandlungsfluid zuführbar ist, **dadurch gekennzeichnet, dass**
das Trennelement (12, 22, 32, 42) wenigstens ein Plattenelement (57) aus einem zumindest elektrisch leitfähigen oder zumindest ferromagnetischen Werkstoff aufweist, welches Plattenelement (57) bei einem Auslenken zumindest eines Bereichs des Trennelements (12, 22, 32, 42) mit ausgelenkt wird, wobei das Plattenelement (57) von einer das Trennelement (12, 22, 32, 42) haltenden Einfassung (58) des Trennelementes (12, 22, 32, 42) beabstandet und in einem zentralen Aufnahmebereich (59) des Trennelementes (12, 22, 32, 42) angeordnet ist, wobei um den Aufnahmebereich (59) ein Ringbereich (60) umläuft, sodass das Trennelement (12, 22, 32, 42) tellerförmig ausgebildet ist.

2. Kassette nach Anspruch 1,
**dadurch gekennzeichnet, dass**
das Plattenelement (57) eine Dicke aufweist, die kleiner als eine Dicke des Trennelements (12, 22, 32, 42) ist.

3. Kassette nach einem der vorhergehenden Ansprüche,
**dadurch gekennzeichnet, dass**
das Plattenelement (57) eine Beschichtung aufweist.

4. Kassette nach einem der vorhergehenden Ansprüche,
**dadurch gekennzeichnet, dass**
das Trennelement (12, 22, 32, 42) antriebskammerseitig wenigstens ein Verbindungselement (61) zum mechanischen Verbinden des im Aufnahmebereich (59) angeordneten Plattenelements (57) mit dem Trennelement (12, 22, 32, 42) aufweist.

5. Kassette nach Anspruch 4,
**dadurch gekennzeichnet, dass**
das wenigstens eine Verbindungselement (61) wenigstens eine Aufnahmenut (62) für einen Rand des Plattenelements (57) aufweist.

6. Konsole (1) eines ophthalmochirurgischen Systems (100) zur Behandlung eines Auges (6), zumindest mit
- einem Kassettenaufnahmebereich (56) zum Aufnehmen einer Kassette (4), die wenigstens eine Fluidpumpe (10, 20, 30, 40) zum Fördern eines Behandlungsfluids aufweist, wobei die Fluidpumpe (10, 20, 30, 40) eine Pumpkammer (11, 21, 31, 41) und eine mittels eines zumindest bereichsweise auslenkbaren Trennelements (12, 22, 32, 42) von der Pumpkammer (11, 21, 31, 41) getrennte Antriebskammer (13, 23, 33, 43) aufweist, wobei der Antriebskammer (13, 23, 33, 43) ein Antriebsfluid und der Pumpkammer das Behandlungsfluid zuführbar ist,
- einem Antriebsfluidversorgungssystem (63) zum Zuführen des Antriebsfluids in die Antriebskammer (13, 23, 33, 43), und
- einer Sensoreinheit (19, 29, 39, 49) zum Erfassen einer Auslenkungsposition des Trennelements (12, 22, 32, 42),
**dadurch gekennzeichnet, dass**
die Sensoreinheit (19, 29, 39, 49) ausgebildet ist, unter Nutzung eines Magnetfeldes eine Auslenkungsposition des am Trennelement (12, 22, 32, 42) angeordneten Plattenelements (57) einer Kassette nach einem der vorhergehenden Ansprüche zu erfassen.

7. Ophthalmochirurgisches System (100) zur Behandlung eines Auges (6), mit zumindest:
- einer in einen Kassettenaufnahmebereich (56) einer Konsole (1) einsetzbaren Kassette (4), die wenigstens eine Fluidpumpe (10, 20, 30, 40) zum Fördern eines Behandlungsfluids aufweist, wobei die Fluidpumpe (10, 20, 30, 40) eine Pumpkammer (11, 21, 31, 41) und eine mittels eines zumindest bereichsweise auslenkbaren Trennelements (12, 22, 32, 42) von der Pumpkammer (11, 21, 31, 41) getrennte Antriebskammer (13, 23, 33, 43) aufweist, wobei der Antriebskammer (13, 23, 33, 43) ein Antriebsfluid und der Pumpkammer ein Behandlungsfluid zuführbar ist,
- der Konsole (1), die den Kassettenaufnahmebereich (56) zum Aufnehmen der Kassette (4), ein Antriebsfluidversorgungssystem (3) zum Zuführen eines Antriebsfluids in die Antriebskammer (13, 23, 33, 43) und eine Sensoreinheit (19, 29, 39, 49) zum Erfassen einer Auslenkungsposition des Trennelements (12, 22, 32, 42) aufweist, und
- einem ophthalmochirurgischen Handstück (5) zum Behandeln einer Augenlinse (7) des Auges (6), welches zumindest mit der Konsole (1) oder der Kassette (4) koppelbar ist,
**dadurch gekennzeichnet, dass**
zumindest die Kassette (4) nach einem der Ansprüche 1 bis 5 oder die Konsole (1) nach Anspruch 6 ausgebildet ist.

## Claims

1. Cartridge (4) for a panel (1) of an ophthalmic surgical system (100) for treating an eye (6), the cartridge (4) being designed for insertion in a cartridge accommodation region (56) of the panel (1) and having at least one fluid pump (10, 20, 30, 40) for conveying a treatment fluid, the fluid pump (10, 20, 30, 40) having a pump chamber (11, 21, 31, 41) and a drive chamber (13, 23, 33, 43) separated from the pump chamber (11, 21, 31, 41) by way of a fluid-tight and ion-tight partition element (12, 22, 32, 42) that is at least regionally deflectable, with a drive fluid being feedable to the drive chamber (13, 23, 33, 43) and the treatment fluid being feedable to the pump chamber,
**characterized in that**
the partition element (12, 22, 32, 42) has at least one plate element (57) made of an at least electrically conductive or at least ferromagnetic material, the plate element (57) also being deflected in the case of a deflection of at least one region of the partition element (12, 22, 32, 42) and the plate element (57) being spaced apart from an edging (58) of the partition element (12, 22, 32, 42) that holds the partition element (12, 22, 32, 42) and being arranged in a central accommodation region (59) of the partition element (12, 22, 32, 42), with a ring region (60) running around the accommodation region (59) such that the partition element (12, 22, 32, 42) is in the form of a plate.

2. Cartridge according to Claim 1,
**characterized in that**
a thickness of the plate element (57) is smaller than a thickness of the partition element (12, 22, 32, 42).

3. Cartridge according to either one of the preceding claims,
**characterized in that**
the plate element (57) has a coating.

4. Cartridge according to any one of the preceding claims,
**characterized in that**
the partition element (12, 22, 32, 42) has at least one connection element (61) located on the drive chamber side and serving for the mechanical connection of the plate element (57) arranged in the accommodation region (59) to the partition element (12, 22, 32, 42).

5. Cartridge according to Claim 4,
**characterized in that**
the at least one connection element (61) has at least one receiving groove (62) for an edge of the plate element (57).

6. Panel (1) of an ophthalmic surgical system (100) for treating an eye (6), at least comprising
- a cartridge accommodation region (56) for accommodating a cartridge (4) having at least one fluid pump (10, 20, 30, 40) for conveying a treatment fluid, the fluid pump (10, 20, 30, 40) having a pump chamber (11, 21, 31, 41) and a drive chamber (13, 23, 33, 43) separated from the pump chamber (11, 21, 31, 41) by way of a partition element (12, 22, 32, 42) that is at least regionally deflectable, with a drive fluid being feedable to the drive chamber (13, 23, 33, 43) and the treatment fluid being feedable to the pump chamber,
- a drive fluid supply system (63) for feeding the drive fluid into the drive chamber (13, 23, 33, 43), and
- a sensor unit (19, 29, 39, 49) for detecting a deflection position of the partition element (12, 22, 32, 42),
**characterized in that**
the sensor unit (19, 29, 39, 49) is designed to use a magnetic field to detect a deflection position of the plate element (57) which is part of a cartridge according to any one of the preceding claims and arranged on the partition element (12, 22, 32, 42).

7. Ophthalmic surgical system (100) for treating an eye (6), at least having:
- a cartridge (4) insertable in a cartridge accommodation region (56) of a panel (1) and having at least one fluid pump (10, 20, 30, 40) for conveying a treatment fluid, the fluid pump (10, 20, 30, 40) having a pump chamber (11, 21, 31, 41) and a drive chamber (13, 23, 33, 43) separated from the pump chamber (11, 21, 31, 41) by way of a partition element (12, 22, 32, 42) that is at least regionally deflectable, with a drive fluid being feedable to the drive chamber (13, 23, 33, 43) and a treatment fluid being feedable to the pump chamber,
- the panel (1) which has the cartridge accommodation region (56) for accommodating the cartridge (4), a drive fluid supply system (3) for feeding a drive fluid into the drive chamber (13, 23, 33, 43) and a sensor unit (19, 29, 39, 49) for detecting a deflection position of the partition element (12, 22, 32, 42), and
- an ophthalmic surgical handpiece (5) for treating a crystalline lens (7) of the eye (6), which is able to be coupled to at least the panel (1) or the cartridge (4), **characterized in that**
at least the cartridge (4) is designed according to any one of Claims 1 to 5 or the panel (1) is designed according to Claim 6.

## Revendications

1. Cassette (4) pour une console (1) d'un système de chirurgie ophtalmologique (100) pour le traitement d'un œil (6), la cassette (4) étant réalisée pour être insérée dans une zone de réception (56) de cassette de la console (1) et comportant au moins une pompe à fluide (10, 20, 30, 40) destinée à refouler un fluide de traitement, la pompe à fluide (10, 20, 30, 40) comportant une chambre de pompage (11, 21, 31, 41) et une chambre d'entraînement (13, 23, 33, 43) séparée de la chambre de pompage (11, 21, 31, 41) au moyen d'un élément de séparation (12, 22, 32, 42) étanche aux fluides et aux ions pouvant être dévié au moins par endroits, un fluide d'entraînement pouvant être amené à la chambre d'entraînement (13, 23, 33, 43) et le fluide de traitement pouvant être amené à la chambre de pompage,
**caractérisée en ce que**
l'élément de séparation (12, 22, 32, 42) comporte au moins un élément de plaque (57) composé d'un matériau au moins électriquement conducteur ou au moins ferromagnétique, lequel élément de plaque (57) est dévié lors d'une déviation d'au moins une zone de l'élément de séparation (12, 22, 32, 42), l'élément de plaque (57) étant espacé d'une monture (58), maintenant l'élément de séparation (12, 22, 32, 42), de l'élément de séparation (12, 22, 32, 42) et étant disposé dans une zone de réception (59) centrale de l'élément de séparation (12, 22, 32, 42), une zone annulaire (60) s'étendant autour de la zone de réception (59) si bien que l'élément de séparation (12, 22, 32, 42) est réalisé en forme d'assiette.

2. Cassette selon la revendication 1,
**caractérisée en ce que**
l'élément de plaque (57) présente une épaisseur qui est inférieure à une épaisseur de l'élément de séparation (12, 22, 32, 42).

3. Cassette selon l'une des revendications précédentes,
**caractérisée en ce que**
l'élément de plaque (57) comporte un revêtement.

4. Cassette selon l'une des revendications précédentes,
**caractérisée en ce que**
l'élément de séparation (12, 22, 32, 42) comporte du côté de la chambre d'entraînement au moins un élément de liaison (61) destiné à relier mécaniquement l'élément de plaque (57) disposé dans la zone de réception (59) à l'élément de séparation (12, 22, 32, 42).

5. Cassette selon la revendication 4,
**caractérisée en ce que**
l'au moins un élément de liaison (61) comporte au moins une rainure de réception (62) pour un bord de l'élément de plaque (57).

6. Console (1) d'un système de chirurgie ophtalmologique (100) pour le traitement d'un œil (6), au moins avec
- une zone de réception (56) de cassette destinée à recevoir une cassette (4), qui comporte au moins une pompe à fluide (10, 20, 30, 40) destinée à refouler un fluide de traitement, la pompe à fluide (10, 20, 30, 40) comportant une chambre de pompage (11, 21, 31, 41) et une chambre d'entraînement (13, 23, 33, 43) séparée de la chambre de pompage (11, 21, 31, 41) au moyen d'un élément de séparation (12, 22, 32, 42) pouvant être dévié au moins par endroits, un fluide d'entraînement pouvant être amené à la chambre d'entraînement (13, 23, 33, 43) et le fluide de traitement pouvant être amené à la chambre de pompage,
- un système d'alimentation en fluide d'entraînement (63) destiné à amener le fluide d'entraînement dans la chambre d'entraînement (13, 23, 33, 43), et
- une unité de capteur (19, 29, 39, 49) destinée à détecter une position de déviation de l'élément de séparation (12, 22, 32, 42),
**caractérisée en ce que**
l'unité de capteur (19, 29, 39, 49) est réalisée pour détecter, en utilisant un champ magnétique, une position de déviation de l'élément de plaque (57) d'une cassette disposé sur l'élément de séparation (12, 22, 32, 42) selon l'une des revendications précédentes.

7. Système de chirurgie ophtalmologique (100) pour le traitement d'un œil (6), avec au moins :
- une cassette (4) pouvant être insérée dans une zone de réception (56) de cassette d'une console (1), qui comporte au moins une pompe à fluide (10, 20, 30, 40) destinée à refouler un fluide de traitement, la pompe à fluide (10, 20, 30, 40) comportant une chambre de pompage (11, 21, 31, 41) et une chambre d'entraînement (13, 23, 33, 43) séparée de la chambre de pompage (11, 21, 31, 41) au moyen d'un élément de séparation (12, 22, 32, 42) pouvant être dévié au moins par endroits, un fluide d'entraînement pouvant être amené à la chambre d'entraînement (13, 23, 33, 43) et un fluide de traitement pouvant être amené à la chambre de pompage,
- la console (1), qui comporte la zone de réception (56) de cassette destinée à recevoir la cassette (4), un système d'alimentation en fluide d'entraînement (3) destiné à amener un fluide d'entraînement dans la chambre d'entraînement (13, 23, 33, 43) et une unité de capteur (19, 29, 39, 49) destinée à détecter une position de déviation de l'élément de séparation (12, 22, 32, 42), et
- une pièce à main (5) de chirurgie ophtalmologique pour le traitement d'un cristallin (7) de l'œil (6), laquelle peut être couplée au moins à la console (1) ou à la cassette (4),
**caractérisée en ce que**
au moins la cassette (4) est réalisée selon l'une des revendications 1 à 5 ou la console (1) est réalisée selon la revendication 6.
